(19) Europäisches Patentamt
European Patent Office
Office européen des brevets

(11) Publication number: 0 408 019 A2

# (12) EUROPEAN PATENT APPLICATION

(21) Application number: 90113294.4

(22) Date of filing: 11.07.90

(51) Int. Cl.5: A61K 9/06, A61K 9/70, A61M 35/00, A61L 15/00

(30) Priority: 12.07.89 US 378838

(43) Date of publication of application: 16.01.91 Bulletin 91/03

(84) Designated Contracting States: AT BE CH DE DK ES FR GB GR IT LI LU NL SE

(71) Applicant: UNION CARBIDE CHEMICALS AND PLASTICS COMPANY, INC.
39 Old Ridgebury Road
Danbury Connecticut 06817-0001(US)

(72) Inventor: Partain III, Emmett Malone
267 Park Avenue
Bound Brook (08805) N.J.(US)
Inventor: Brode II, George Lewis
653 Carlene Drive
Bridgewater (08807) N.J.(US)

(74) Representative: Weinhold, Peter, Dr. et al
Patentanwälte Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

## (54) Pharmaceutical active derivative delivery systems.

(57) A biocompatible, film-forming delivery system for the delivery of pharmaceutical or therapeutic actives to a desired topical site of a subject, the system including:
an active derivatized compound, wherein the active derivatized compound is a reaction product of
(1) a cyclic anhydride moiety-containing. biocompatible, film-forming polymer with
(2) at least one active selected from the group consisting of pharmaceutical actives, therapeutic actives and a combination thereof, wherein the at least one active has a moiety selected from the group consisting of (a) a hydroxyl moiety capable of forming a half-ester reaction product with a cyclic anhydride moiety of the polymer, (b) an amine moiety capable of forming a half-amide reaction product with a cyclic anhydride moiety of the polymer, and (c) a combination thereof, and
wherein the active derivatized compound forms an active derivatized compound film on the site and the active is released from the active derivatized compound while the delivery system is in contact with the site such that the polymer remains thereon as a polymer film and the at least one active is delivered to the site in an absorbable form.



Xerox Copy Centre

# PHARMACEUTICALLY ACTIVE DERIVATIVE DELIVERY SYSTEMS

## FIELD OF THE INVENTION

This invention relates, in general, to novel delivery systems useful for the topical delivery of pharmaceutical or therapeutic actives. In one aspect, this invention relates to delivery systems containing certain derivatized compounds of a cyclic anhydride moiety-containing, biocompatible polymer derivatized with such actives which are effective systems for the delivery of a variety of such pharmaceutical and therapeutic actives. In a further aspect, this invention is directed to the preparation and use of such systems.

## BACKGROUND OF THE INVENTION

Traditionally, pharmaceutical and therapeutic actives can be administered to the body by a number of routes, including ingestion, injection, inhalation, and topical application. Absorption of an active by ingestion, injection, or inhalation generally gives systemic distribution of the active throughout the body. Systemic distribution of the active may be unsatisfactory for three reasons. First, these modes of administration produce non-specific distribution. The active is distributed through the entire body and not localized. Second, there may be undesirable effects such as toxic or irritating reactions on non-target organs or regions. Finally, to achieve the desired effect at the target organ or region, a higher dosage than might otherwise be desired must be administered to compensate for systemic dilution of the active.

In contrast to systemic delivery, topical delivery is application of an active in a manner so that it acts primarily at the site of application. The above-described deficiencies of systemic delivery are not encountered when an active is applied topically. Rather, topical application affords the opportunity to minimize the dosage and confine the active to the region of the body to which it is applied. Thus systemic distribution of the active throughout the body is obviated. Typical sites of topical delivery include application to the dermal, opththalmic and mucous membranes and tissues, such as the hair, skin, eyes, ears, mouth, nose, throat, rectum, vagina, and urethra.

However, despite these advantages of topical delivery, most current topical delivery formulations are inefficient and therefore have limited utility. There are three reasons for this inefficiency of current topical delivery technology. First, skin and mucous membranes possess good barrier properties and the permeability of most actives through these barriers generally is poor. Second, actives applied topically are subject to migration and loss due to perspiration, natural tissue lavation, and mechanical action particularly because such actives are not substantive, not readily absorbed by the skin, and do not form films. Third, because most pharmaceutical or therapeutic actives are relatively simple, low molecular weight compounds or mixtures, these actives have limited solubility in common solvents such as water and alcohol. The actives tend to crystallize and flake-off the skin, for example, before they can be absorbed.

Consequently, considerable effort has been and is being expended in search of a proper delivery system which can minimize undesirable crystallization of the active, deliver the active to the application site, control the dosage thereof, and optimize its availability in its active form. Most known topical delivery systems are petrolatum-based cremes and ointments. These unctuous formulations are unsatisfactory because they are at best uncomfortable and messy when applied to skin and mucous membrane (mucosa).

A topical delivery system cannot be considered fully satisfactory if it is deficient with regard to any of the above-described criteria. For example, a delivery system which does not ensure that the active efficiently penetrates the application site is not satisfactory because it requires that an excess of active be incorporated into the delivery system to ensure delivery of an effective quantity. The remaining active, i.e., that which does not penetrate the application site, is wasted. Similarly, active which is allowed to migrate from the application site, or to crystallize before it penetrates the site, is wasted. Further, a delivery system which satisfies each criterion will be adjudged a failure by a consumer who is dissatisfied because the delivery system leaves an unpleasant residue. For example, an unctuous residue, which is unpleasant to the touch and messy, may cause a consumer not to utilize the treatment. Thus, such delivery systems are unsatisfactory.

## SUMMARY OF THE INVENTION

Accordingly, the present invention is directed to novel delivery systems comprised of certain derivatized compounds of a cyclic anhydride moiety-containing biocompatible polymer derivatized with a pharmaceutical or therapeutic active. Optionally, the delivery system may also include a delivery enhancer. The invention also relates a method for preparing the delivery systems, and to a method for their application to a subject.

The delivery system of the present invention is a biocompatible, film-forming delivery system for the delivery of pharmaceutical or therapeutic actives to a desired topical site of a subject or host. The system comprises:

an active derivatized compound, wherein the active derivatized compound is a reaction product of

(1) a cyclic anhydride moiety-containing biocompatible, film-forming polymer with

(2) at least one active selected from the group consisting of pharmaceutical actives, therapeutic actives and a combination thereof, wherein the at least one active has a moiety selected from the group consisting of (a) a hydroxyl moiety capable of forming a half-ester reaction product with a cyclic anhydride moiety of the polymer (b) an amine moiety capable of forming a half-amide reaction product with a cyclic anhydride moiety of the polymer, and (c) a combination thereof, and

wherein the active derivatized compound forms an active derivatized compound film on the site and the active is released from the active derivatized compound while the delivery system is in contact with the site such that the polymer remains thereon as a polymer film and the at least one active is delivered to the site in an absorbable form.

The system efficiently delivers the actives to the user at the application site and provides at the site a non-irritating, essentially imperceptible, gas permeable film over the application site.

## IN THE DRAWINGS

Figure 1 is a graph showing the progress of lesion scores during a cutaneous model using Nude mice.

## DETAILED DESCRIPTION OF THE INVENTION

This invention is based on the discovery that a delivery system for pharmaceutical or therapeutic actives comprising:

an active derivatized compound, wherein the active derivatized compound is a reaction product of

(1) a cyclic anhydride moiety-containing, biocompatible, film-forming polymer with

(2) at least one active selected from the group consisting of pharmaceutical actives, therapeutic actives and a combination thereof, wherein the at least one active has a moiety selected from the group consisting of (a) a hydroxyl moiety capable of forming a half-ester reaction product with a cyclic anhydride moiety of the polymer (b) an amine moiety capable of forming a half-amide reaction product with a cyciic anhydride moiety of the polymer and (c) a combination thereof, and

wherein the active derivatized compound forms an active derivatized compound film on the site and the active is released from the active derivatized compound while the delivery system is in contact with the site such that the polymer remains thereon as a polymer film and the at least one active is delivered to the site in an absorbable form;

provides unexpectedly efficient delivery of the active. Optionally, a delivery enhancer may be added to further enhance the efficiency of the delivery of the active to the application site.

As used throughout the specification and claims, the phrase "pharmaceutical active" is considered to be a drug, i.e., a substance which, when applied to or introduced into the body, alters body functions in some way. The phrase "therapeutic active" is broader in scope and includes any substance which is capable of altering either body function or cosmetic appearance, but which is not traditionally or technically considered a drug. For example, mineral oil does alter the skin in at least a cosmetic manner or in some cases may be therapeutic. Therefore, mineral oil is considered to be a "therapeutic active" for purposes of the present invention.

There are several features which make the delivery systems of the present invention superior delivery vehicles. In the first instance, the delivery systems of this invention are preferably substantive with hair, skin, and mucous membrane. Throughout the specification and claims, the term "substantive" means that there exists a cohesive or adhesive interaction between the anhydride moiety-containing, biocompatible polymer and the polycarboxylic acid thereof and a proteinaceous substrate, i.e., the hair, skin, or mucosa, to which the delivery system is applied. In the delivery systems of the present invention, substantivity typically is obtained by ensuring that the polymer is amphoterically or hydrophobically modified. Incorporation of

appropriate hydrophobic groups, amphoteric groups, and combinations thereof provide substantivity.

Hydrophobic groups may be incorporated into the polymer by derivatizing the cyclic anhydride moieties of the polymer with a primary, secondary or tertiary alcohol or a primary or secondary amine to give the half-ester or half-amide derivatives of the polymer, respectively. If a polycarboxylic acid polymer is utilized, at least a portion of the carboxylic acid moieties thereof are first converted to their corresponding cyclic anhydride moieties.

The alcohols and amines preferably have at least eight carbons therein. The alcohols may be alkyl alcohols, alkaryl alcohols and aralkyl alcohols, preferably alkyl alcohols. The amines are preferably alkyl amines. Dodecyl alcohol and dodecyl amine are examples of an alkyl alcohol and an alkyl amine, respectively.

The polymer may be amphoterically modified reacting the polymer with cationic alcohols, for example, choline chloride, and/or quaternary compounds, for example, hydroxy propyl trimethyl ammonium chloride. Thus, the delivery systems of the present invention may be tailored to exhibit a cohesive interaction with the proteins of hair, skin, and mucosa.

The cyclic anhydride moiety-containing, biocompatible polymers and active derivative thereof utilized in the delivery system of the present invention are those which are good film-formers, i.e., a polymeric film is readily formed when a solution of these polymers and/or active half-esters in a biocompatible solvent is applied topically. Upon topical application of the delivery system of this invention, a polymeric film forms and the active derivative thereof (active derivatized compound) serves as a reservoir from which the active is continuously delivered. The film also serves to protect the application site from insult or injury.

Biocompatible solvents are well-known by those skilled in the art. Water, ethanol, propylene glycol, hexylene glycol, glycerine. and mixtures thereof are the most commonly used biocompatible solvents. Additionally, such biocompatible solvents may be utilized which provide other desirable functions in a delivery system. For example. pyrrolidone carboxylic acid (PCA) is also an effective moisturizing agent which has a low order of irritation. Such secondary functions of biocompatible solvents are also well-known by those skilled in the art.

The hydrophobically and/or amphoterically derivatized polymer exhibit substantive properties to keratin and other protein constituents of hair, skin, and mucosa. Thus, upon application of these materials to these tissues, the resulting film is bound to the tissue. This close relationship minimizes loss or migration of the film and the active. Any form of the delivery system, such as a lotion (solution of a non-aqueous fraction and an aqueous fraction), creme or ointment (both emulsions), spray (aerosol or powder, for example) comprising the active derivatized compound conveniently may be utilized to form the subject delivery system. The system may also be applied to the skin or mucosa in the form of a pre-formed film, sponge, powder or other composite, as described below.

Application of an active derivatized compound-containing delivery system which forms a film provides uniform distribution of the active on the tissue and prevents migration or loss of the active from the site of application. The reservoir of active in the film helps to control the rate of release. The biocompatible polymers and active derivatized compounds of the present invention are those which do not elicit an inflammatory, allergic, or pyrogenic response in humans after injestion or percutaneous or subcutaneous application. In addition, the films these materials form on skin and mucosa are preferably selected so as to be essentially imperceptible to the patient and cosmetically comfortable to wear. The making of such selections is within the capabilities of one skilled in the art without undue experimentation.

The cyciic anhydride molety-containing, biocompatible, film-forming polymers utilized herein are selected from the class of cyclic anhydride moiety-containing polymers and from the class of polycarboxylic acid polymers wherein the carboxylic acid moieties thereof may be converted at least in part to their corresponding cyclic anhydride moieties. The polymer may be a homopolymer, copolymer or graft polymer. The homopolymer is a polymer of an ethylenically unsaturated mono-carboxylic acid wherein at least a portion of the carboxylic acid moieties thereof in pairs may be converted to their corresponding cyclic anhydride moiety, or an ethylenically unsaturated poly-carboxylic acid or cyclic anhydride thereof. The copolymer is a polymer of an ethylenically unsaturated mono-carboxylic acid and/or an ethylenically unsaturated poly-carboxylic acid or cyclic anhydride thereof copolymerized with one or more ethylenically unsaturated monomers which do not have carboxylic acid mole ties or cyclic anhydride moieties. The graft polymer is a homopolymer or copolymer of at least one ethylenically unsaturated monomer to which is grafted at least one oligomer of an ethylenically unsaturated mono-carboxylic acid wherein the oligomer is capable of forming at least one cyclic anhydride moiety, or at least one ethylenically unsaturated poly-carboxylic acid or cyclic anhydride thereof. The homopolymer and copolymer may be the product of polymerizing one or more conjugated-dienes, such as butadiene or isoprene, which are then selectively hydrogenated leaving a residual amount of ethylenic unsaturation therein at which ethylenically unsaturated

carboxylic acids and/or ethylenically unsaturated cyclic anhydrides may be grafted. Generally, polymerization and hydrogenation are carried out in solution with a suitable catalyst therefor. The grafting reaction may take place in solution or in the melt, such as in an extruder. Such polymerization, copolymerization and grafting processes and methods are well-known to those skilled in the art, as well as selective hydrogenation.

The ethylenically unsaturated carboxylic acids and cyclic anhydrides utilized in the polymers hereof preferably have 2 to about 10 carbon atoms excluding those in the carboxyl and/or cyclic anhydride groups thereof.

Examples of such ethylenically unsaturated mono-carboxylic acids include, but are not limited to, acrylic acid, methacrylic acid, crotonic acid, and the like. Oligomers or polymer sequences of these monomers may be capable of forming a cyclic anhydride which may be utilized to incorporate the active and/or hydrophobic moieties into the polymer to cationically-charge the polymer.

The ethylenically unsaturated poly-carboxylic acids and cyclic anhydrides thereof are preferably ethylenically unsaturated dicarboxylic acids and cyclic anhydrides thereof and more preferably alpha, beta-ethylenically unsaturated dicarboxylic acids and cyclic anhydrides thereof.

Examples of such ethylenically unsaturated poly-carboxylic acids and cyclic anhydrides thereof include, but are not limited to, maleic acid, fumaric acid, maleic anhydride, itaconic acid, itaconic anhydride, citraconic acid, mesaconic acid, citraconic anhydride, aconitic acid (a tricarboxylic acid), aconitic anhydride, cis-4-cyclohexene-1,2-dicarboxylic acid, cis-4-cyclohexene-1,2-dicarboxylic anhydride, endo-cis-bicyclo (2,2,1)-5-heptene-2,3-dicarboxylic acid, and ende-cis-bicylco (2,2,1)-5-heptene-2,3-dicarboxylic anhydride. These modifiers may be used alone or in combination thereof. Among these ethylenically unsaturated poly-carboxylic acids and cyclic anhydrides thereof, maleic acid, fumaric acid and maleic anhydride are particularly preferred, with maleic anhydride most preferred.

Examples of ethylenically unsaturated non-carboxylic monomers include, but are not limited to, vinyl ethers, vinyl esters, vinyl amides, and olefins.

Examples of vinyl ethers include, but are not limited to, vinyl methyl ether, vinyl dodecyl ether, divinyl ether, and vinyl isopropyl ether.

Examples of vinyl esters include, but are not limited to, vinyl acetate, vinyl stearate, and vinyl laurate.

Examples of vinyl amides include, but are not limited to, N-vinyl pyrrolidone.

Examples of olefins include, but are not limited to, ethylene, propylene, styrene, acrylonitrile vinyl imidazole, vinyl pyridine and conjugated-dienes, for example, butadiene and isoprene.

Additionally, these copolymers may be block, tapered, random or regularly alternating copolymers. Again such copolymerization processes and methods and resulting polymers are well-known to those skilled in the art. An example of such block copolymers are HYPAN® polymers available from Kingston Technologies of Dayton, New Jersey, which include block copolymers of acrylic acid and acrylonitrile according to U.S. Patent No. 4,420,589. An example of such regularly alternating copolymers are UCARSET ® polymers available from Union Carbide Co., which include regularly alternating copolymers of vinyl methyl ether and maleic anhydride.

In general, the amount of the active derivatized compound employed in the compositions of this invention will vary depending upon the particular pharmaceutical or therapeutic active being delivered, whether a diluent is present, the type of additives, and the like. In practice, however, it has been found that a concentration of the active derivatized compound in the composition can range up to about 30, preferably between about 0.05 and about 10, weight percent. based on the total weight of the composition of the delivery system.

The delivery systems of the present invention contain pharmaceutical and therapeutic actives that can be applied topically either singularly or in combination. Examples of these actives include, but are not limited to, compounds such as acyclovir (antiviral agent), retinol (vitamin derivative), retinoic acid (both cis and trans; vitamin derivative), 7-dehydrocholesterol (vitamin D), benzoctamine (muscle relaxant), and the like.

As indicated above, this list of pharmaceutical and therapeutic actives is not inclusive, but is presented merely to demonstrate the scope of the invention. A wide variety of other actives can be employed either alone or in combination. The only requirement is that these actives be capable of forming the active derivatized compound hereof.

Thne amount of active employed will be that amount necessary to deliver a pharmaceutically or therapeutically effective amount to achieve the desired result at the site of application. In particular, an effective amount depends, inter alia , upon the particular active, the severity of the condition, and other factors. In general. the concentration of the actives in the delivery systems can vary from as little as 0.01 up to 50 percent or higher, by weight of the delivery system. More typically, the active concentration is

between about 0.1 and about 20 wt percent of the delivery system. Skilled practitioners will be able to adjust the quantity of active in the delivery system.

The delivery system of the present invention is particularly applicable to the delivery of actives which have limited solubility in biocompatible solvents and have a tendency to crystallize prior to absorption thereof into the applied area and then flake-off. In the active derivatized compound hereof, the active is covalently bound to the active derivatized compound on a molecular level. As such, crystallization of the active is prevented, or at least significantly inhibited, especially when applied to the application site.

Take for example a crystallizing active of interest such as acyclovir. The acyclovir is covalently bound to the cyclic anhydride moiety-containing, biocompatible polymer after having reacted with a cyclic anhydride moiety of the polymer, thereby forming the active derivatized compound. In this case. the active derivatized compound is an active half-ester.

Acyclovir has the following structure:

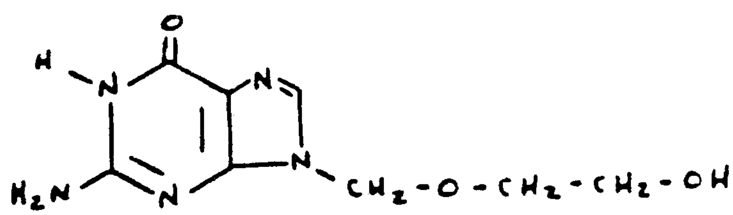

The structure thereof may be simplified for the purpose of illustration to that of a primary alcohol; i.e. R-$CH_2$-OH. Actives which are primary, secondary, or tertiary alcohols and reactive in the following manner may also be utilized in the present invention.

For illustrative purposes, the cyclic anhydride moiety-containing, biocompatible polymer may be structurally simplified and represented as follows:

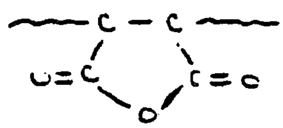

where the wavy line represents the backbone of the polymer.

The cyclic anhydride moiety-containing biocompatible polymer and the acyclovir react in the following manner to form a half-ester, thereby forming the active derivatized compound. (acyclovir) (polymer) (active derivatized compound)

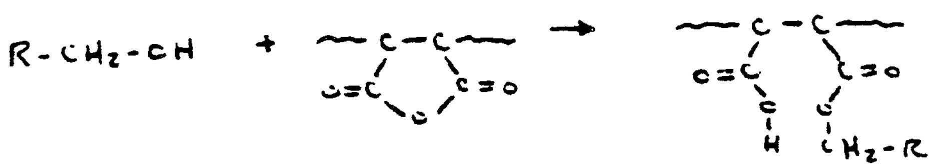

Thus as is readily apparent, the active, in this example acyclovir, is covalently bound to the polymer on a molecular level.

Another crystallizing active of interest is benzoctamine (N-methyl-9,10-ethanoanthracene-9(10H)-methanamine. The benzoctamine is also covalently bound to the cyclic anhydride moiety-containing, biocompatible polymer after having reacted with a cyclic anhydride moiety of the polymer, thereby forming the active derivatized compound. In this case, the active derivatized compound is an active half-amide.

Benzoctamine has the following structure:

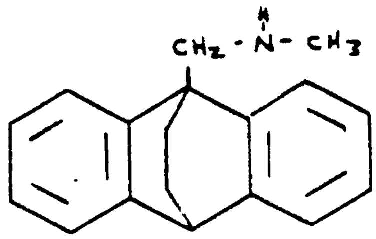

The structure thereof may be simplified for the purpose of illustration to that of a secondary amine; i.e. H-N(R)$_2$. Actives which are primary or secondary amines and reactive in the following manner may also be utilized in the present invention.

The cyclic anhydride moiety-containing, biocompatible polymer and the benzoctamine would react in the following manner to form a half-amide, thereby forming the active derivatized compound. (benzoctamine) (polymer) (active derivatized compound)

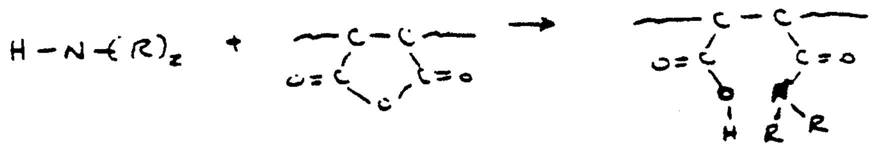

Thus, as is readily apparent, the active, in this example benzoctamine, would also be covalently bound to the polymer on a molecular level.

Though acyclovir may be considered both a primary alcohol and a primary amine, IR analysis of an acyclovir derivatized vinyl methyl ether/maleic anhydride copolymer revealed ester moieties and not amide moieties. Though not wishing to be bound to any particular theory, it is believed that both the hydroxyl and amine moieties therein are competing for the maleic anhydride moieties and that the hydroxyl moiety is more reactive than the amine moiety therein.

Once the delivery system is applied to the subject, the active a released in an absorbable form and delivered to the application site. The exact nature of this release of the active from the active derivatized compound is not entirely understood. However, it is known that the active is released while the delivery system is in contact with the application site of the subject. As such, the environment of the application site appears to be conducive to the release of the active. As the active is released, the initial anhydride moiety to which the active had been bound is believed to have been converted to its corresponding dicarboxylic acid form. Crystallization of the active has not been observed; and the active is believed to be released in a molecular form and delivered to the application site in an absorbable form.

In addition to preventing, or at least inhibiting, crystallization of crystallizable actives, covalently bonding the active to the biocompatible polymer may also enhance the substantivity of the delivery system if the active imparts hydrophobic and/or amphoteric qualities to the polymer.

The delivery system of the present invention may optionally also contain a delivery enhancer. These delivery enhancers enhance the delivery of actives which have limited solubility in water through a hydrophobic surface such as skin.

Examples of such delivery enhancers include, but are not limited to, benzyl alcohol, benzoate of $C_{12}$-$C_{15}$ alcohols, vaccenic acid (cis-octadecen-11-oic acid), hexylene glycol, azone, and dimethyl sulfoxide (DMSO).

If desired, the delivery systems of this invention can contain one or more pharmaceutically acceptable diluents or vehicles in addition to the active derivatized compound and the delivery enhancer. In many instances, the active derivatized compound itself can about 0.5 to about 90 weight percent of the system with the remainder being diluent and optionally, other additives. Suitable diluents include water and among others, ethanol, isopropanol glycerine, propylene glycol, polyethylene giycol, hexylene glycol, thoxylated or propoxylated glucose, sorbitol derivatives, and the like, and aqueous mixtures thereof, and combinations thereof.

Additives for the enhanced percutaneous absorption of various pharmaceutical or therapeutic actives also may be utilized. Such percutaneous enhancers include propylene giycol, glycerol, urea, diethyl sebecate, sodium lauryl sulfate, sodium laureth sulfate, sorbitan ethoxylates, nicotinate esters (such as hexyl nicotinate), oleic acid, pyrrolidone carboxylate esters, (such as dodecyl pyrrolidone carboxylate), N-methyl pyrrolidone, N,N-diethyl-m-toluamide, dimethyl sulfoxide, decyl methyl sulfoxide, alkyl methyl sulfoxides, N,N-dimethyl formamide, cis -11-octadecenoic acid, 1-dodecylazacycloheptan-2-one, and 1,3-dioxacyclopentane or 1,2-dioxacyclohexane containing at least one aliphatic group of four to eighteen carbon atoms.

Known methods for topically delivering certain actives typically require repeated applications during a 24-hour period to ensure that a sufficient quantity of active is delivered to the site. Repeated application is at best inconvenient, and at worst lead to uneven treatment, such as lack of treatment because the scheduled application time fell during a period of sleep, or at a time when application was impossible.

Repeated application of known topical treatments is necessary because the treatments cannot deliver a sufficient quantity of active in one application, e.g.. without feeling greasy or delivering the active at an uneven rate. However, the delivery system of the invention affords an even delivery rate over a long period.

Delivery systems of the invention typically comprise lotions or oil-in-water emulsions. Oil-in-water emulsions feel relatively "non-greasy" when applied, whereas water-in-oil emulsions tend to have a greasy or oily feel. Therefore, oil-in-water emulsions are preferred by consumers.

Emulsion-type delivery systems of the invention are made by the "direct" method or by the "inversion" method. In the "direct" method, the oil phase is dispersed into the continuous aqueous phase to form the oil-in-water emulsion directly. An oil-in-water emulsion is made by the "inversion" method by emulsifying the aqueous phase into a continuous oil phase. At first, a water-in-oil emulsion is formed, but, as the quantity of aqueous phase is increased, the emulsion becomes "inverted" and forms an oil-in-water emulsion. Either preparation method can be used to prepare emulsion-type delivery systems of the invention.

The delivery enhancer also can act as an emulsifier between the oil phase and the aqueous phase. Addition of such an emulsifying delivery enhancer bef ore the emulsion is formed typically causes the drop size of the oil phase to be smaller. Smaller drop size may contribute to increasing delivery efficiency.

The delivery enhancers of the invention are distinct from the above-described percutaneous enhancers. The percutaneous enhancers, typically act as humectants, lubricants, softening agents, moisturizers, debris removers, and impart cleansing and other effects. These enhancers therefore prepare the application site to receive active by ensuring that the site is softened, free of debris, and "amenable" to penetration.

In contrast, the delivery enhancers of the subject invention do not provide such functionalities. Rather, the delivery enhancers of the invention serve to provide a path or bridge through the skin, reduce the hydrophobicity of the skin, or otherwise delivering the active more efficiently by reducing the mutual repulsion of hydrophobic skin and hydrophobic actives (such as steroids).

The quantity of delivery enhancer should exceed the minimum quantity which will produce delivery enhancement. Addition of an excess quantity is not economically efficient. Therefore, the quantity of delivery enhancer is up to about 20 wt percent of the delivery system, preferably between about 0.25 and about 10 wt percent, and more preferably between about 0.5 and about 5 wt percent.

In practice, the delivery systems of the invention are readily formulated by mixing a non-aqueous fraction containing at least one delivery enhancer with a solution or suspension of the active derivatized compound. rne solution or suspension of the active derivatized compound may be another non-aqueous fraction or an aqueous fraction depending upon the particular application and whether a suitable non-aqueous biocompatible solvent is available for the compound.

If the active derivatized compound is in an aqueous phase, an emulsion is formed in the non-aqueous fraction is not soluble in the aqueous phase together with a suitable emulsifier. Other adjuvant ingredients such as glycerine, propylene glycol, sorbitol, preservatives, stearic acid, cetyl alcohol, other high molecular weight alcohols, surfactants, menthol, eucalyptus oil, other essential oils, fragrances, penetration enhancers, and the like may be utilized to give stable delivery systems, such as cremes, ointments, lotions, and aerosols, may also be included.

Alternatively, solutions or mixtures of the active salt compound may be fabricated into films, rods, sheets, sponges, or fibers for use as suppositories, medicated sutures, medicated sheets, medicated bandages, patches, and the like.

The following examples are for illustrative purposes only and are not meant to limit the claimed invention in any manner.

For illustrative purposes only, the following examples utilize a copolymer of vinyl methyl ether and maleic anhydride. The specific polymer utilized is UCARSET polymer (DP-100) avallable from Union Carbide Corporation. The UCARSET polymer (DP-100) is a copolymer of an ethlenically unsaturated carboxylic acid or cyclic anhydride thereof and a vinyl ether, more specifically a regularly alternating copolymer of vinyl methyl ether and maleic anhydride having a molecular weight of greater than about 10,000 and is in a solid, powdered form. The family of UCARSET polymers are currently accepted and

used as hair setting resins and denture adhesives.

## EXAMPLES

Throughout the Examples, all parts are parts by weight, unless otherwise identified

Example 1: Preparation of an acyclovir derivatized compound (16303-83)

A 50 ml, single-necked round bottom flask was charged with 0.40 g of acyclovir, 1.50 g of UCARSET DP-100 polymer, and 23.1 g of N-methyl pyrrolidone. The flask was fitted with a magnetic stirring bar and a drying tube with anhydrous calcium sulfate (drying agent). The solid completely dissolved, and the mixture was stirred while heating at 70° C with a water bath for 12 hours.

The polymer (acyclovir derivatized compound) designated Polymer I was recovered by non-solvent precipitation from 1000 ml of diethyl ether. The fluffy precipitated polymer (acyclovir derivatized compound) was dried in vacuo at ambient temperature over phosphorous pentoxide yielding 2.02 g of product containing 18.6% by weight acyclovir (theoretical acyclovir content of 21.1%). A 9% solution of acyclovir derivatized UCARSET polymer was prepared by mixing 0.90 g of acyclovir derivatized UCARSET polymer with 9.1 g of propylene glycol in a water bath at 70° C until the mixture was homogeneous (about 5 to 10 minutes). This clear, colorless solution of acyclovir derivatized UCARSET polymer in propylene glycol can be applied topically to treat a variety of viral diseases.

Example 2: Preparation of an acyclovir derivatized compound (16737-42)

A 3000 ml flask was charged with 68.0 g of acyclovir, 126.31 g of UCARSET DP-100 polymer. and 2000 ml of N-methyl pyrrolidone. The flask was equipped with an overhead stirrer, a nitrogen inlet, and a nitrogen outlet connected to a bubbler. The solid completely dissolved, and the mixture was stirred under nitrogen while heating at 70° C oth an oil bath overnight (about 12 hours).

The polymer (acyclovir derivatized compound) was recovered by non-solvent precipitation using diethyl ether, though toluene may also be used. To avoid cross-linking of the derivatized polymer during coagulation via anhydride formation, water or ethanol, preferably water, was added to the polymer solution.

In the present example, about 10% by volume water or ethanol was added to the polymer solution prior to commencing recovery of the polymer. This solution was stirred at 50° C for about 6 to 7 hours, and allowed to cool over night while continuing to stir same. The solution to which water was added was clear, and the solution to which ethanol was added was not perfectly clear.

Recovery of the polymer was effected by using 50 ml aliquots of the polymer solution/water or ethanol mixture in 1800 ml of diethyl ether. The water containing mixture yielded a very light, fine red-colored powder having no trace of any N-methyl pyrrolidone therein. This polymer is designated Polymer II. The creams of Example 6 using Polymer II recovered from the water containing polymer solution was used "as is" due to the fineness thereof, i.e., no crushing was required.

Polymer II contained about 31% by weight acyclovir (theoretical acyclovir content of 35%).

Example 3: Preparation of an acyclovir derivatized UCARSET polymer in methyl pyrrolidone carboxylate (16464-30)

A 10 ml, single-necked round bottomed flask was charged with 0.0448 g of acyclovir, 0.1545 g of UCARSET DP-100 polymer, and 2.31 g of methyl pyrrolidone carboxylate. The flask was sealed with a rubber serum cap and fitted with a drying tube and magnetic stirring bar. The mixture was heated in a water bath at 75° C for 14 hours, then at 95° C for 9 hours to give a crystal clear, orange fluid.

Methyl pyrrolidone carboxylate is a biocompatible solvent that enhances the absorption of pharmaceuticals through the dermis. The use of methyl pyrrolidone carboxylate (or other pyrrolidone carboxylate esters) as the reaction solvent for the preparation of acyclovir derivatized in UCARSET polymer obviates the need to isolate the polymer by non-solvent precipitation, as described in Examples 1 and 2. The polymer solution can be applied to the dermis "as is".

Example 4: Acyclovir derivatized UCARSET polymer in an aerosol formulation

The acyclovir derivatized UCARSET polymer (Polymer I) in propylene glycol, described in Example 1, was combined with dimethyl ether propellant in an aerosol formulation, which could be used for spray-on applications.

Example 5: Acyclovir derivatized UCARSET polymer in a cream formulation (16804-24 and 25)

Creams I and II were prepared using Polymer II of Example 2 and an acyclovir concentration of about 5% and about 2.5% by weight based on the cream composition, respectively.

Polymer II screened through a 100 mesh screen was dissolved in an 85% glycerol in water mixture, wherein Polymer II constituted about 19.5% by weight based on the total weight of the solution. The solution was stirred and heated at 750° C for about 1 hour and then maintained at 60° C overnight (about 16 hours) while stirring to dissolve Polymer II therein. A minimal number of specs of undissolved polymer remained. This solution is designated Solution A.

Creams I and II were formulated as shown in Table 1.

TABLE 1

| Formulation (grams) | Cream I (5 wt % active) | Cream II (2.5 wt % active) |
|---|---|---|
| Solution A | 276.96 | 138.48 |
| 85% glycerol/water | -- | 138.48 |
| Myrj 52[a] | 9.68 | 9.68 |
| Stearyl alcohol | 12.34 | 12.34 |
| Mineral Oil (Light) | 13.13 | 13.13 |
| Petrolatum white | 13.13 | 13.13 |
| Benzyl alcohol | 3.26 | 3.26 |
| Total | 328.50 | 328.50 |

a. Polyoxyethylene 40 stearate available from ICI Americas, Inc.

The effectiveness of Creams I and II were tested in an Animal model, a cutaneous model using Nude mice. The snouts of the mice were infected with an acycloxir-sensitive strain of herpes simplex virus. Three days after inoculation with the virus, the mice were treated topically three times per day for five days and lesion scores were recorded daily. The study was ended seven days after inoculation. Figure 1 is a graph showing the progress of lesion scores during the experiment with lines "A", "B", "C" and "D" representing the progress of no treatment, the placebo. Cream I and Cream II, respectively.

As is readily apparent from Figure 1, the delivery system of the present invention was effective and did release the acyclovir.

It will be apparent from the foregoing that many other variations and modifications may be made in the processes and the compositions hereinbefore described, by those having experience in this technology, without departing from the concept of the present invention. Accordingly, it should be clearly understood that the processes and compositions referred to herein in the foregoing description are illustrative only and are not intended to have any limitations on the scope of the invention.

## Claims

1. A biocompatible, film-forming delivery system for delivery of pharmaceutical or therapeutic actives to a desired topical site of a subject, said system comprising:
an active derivatized compound, wherein said active derivatized compound is a reaction product of
(1) a cyclic anhydride moiety-containing, biocompatible, film-forming polymer with
(2) at least one active selected from the group consisting of pharmaceutical actives, therapeutic actives and a combination thereof, wherein said at least one active has a moiety selected from the group

consisting of (a) a hydroxyl moiety capable of forming a half-ester reaction product with a cyclic anhydride moiety of said polymer, (b) an amine moiety capable of forming a half-amide reaction product with a cyclic anhydride moiety of said polymer, and (c) a combination thereof, and

wherein said active derivatized compound forms an active derivatized compound film on said site and said active is released from said active derivatized compound while said delivery system is in contact with said site such that said polymer remains thereon as a polymer film and said at least one active is delivered to said site in an absorbable form.

2. The delivery system of claim 1 wherein said polymer is a homopolymer, a copolymer of an ethylenically unsaturated carboxylic acid or cyclic anhydride thereof and a vinyl ether or a graft polymer, said graft polymer having at least one cyclic anhydride moiety or acid thereof grafted thereto.

3. The delivery system of at least one of the claims 1-2 wherein said active has a tertiary amine moiety capable of complexing with at least one carboxylic acid moiety of said polymer to form said active salt compound.

4. The composition of at least one of the claims 1-3 which is in a form selected from the group consisting of a gel, a solution, a lotion, a cream, and an ointment.

5. The composition of at least one of the claims 1-3 which is in the form of a film, a patch, an aerosol, a suppository, a fiber, a rod, or of microspheres.

6. The composition of at least one of the claims 1-3 which is a device selected from the group consisting of a pad and sponge.

7. A method for the preparation of a delivery system for use in administration of pharmaceutical and therapeutic actives to a topical site of a subject, said delivery system comprising:

an active derivatlzed compound, wherein said active derivatized compound is a reaction product of

(1) a cyclic anhydride moiety-containing, biocompatible, film-forming polymer with

(2) at least one active selected from the group consisting of pharmaceutical actives, therapeutic actives and a combination thereof, wherein said at least one active has a moiety selected from the group consisting of (a) a hydroxyl moiety capable of forming a half-ester reaction product with a cyclic anhydride moiety of said polymer, (b) an amine moiety capable of forming a half-amide reaction product with a cyclic anhydride moiety of said polymer and (c) a combination thereof, and

wherein said active derivatized compound forms an active derivatized compound film on said site and said active is released from said active derivatized compound while said delivery system is in contact with said site such that said polymer remains thereon as a polymer film and said at least one active is delivered to the site in an absorbable form.

8. The method of claim 7 which includes a diluent.

9. The method of claim 7 wherein said polymer is dissolved in a solvent before blending.

10. The method of claim 7 wherein said active is dissolved or dispersed in a solvent before blending.

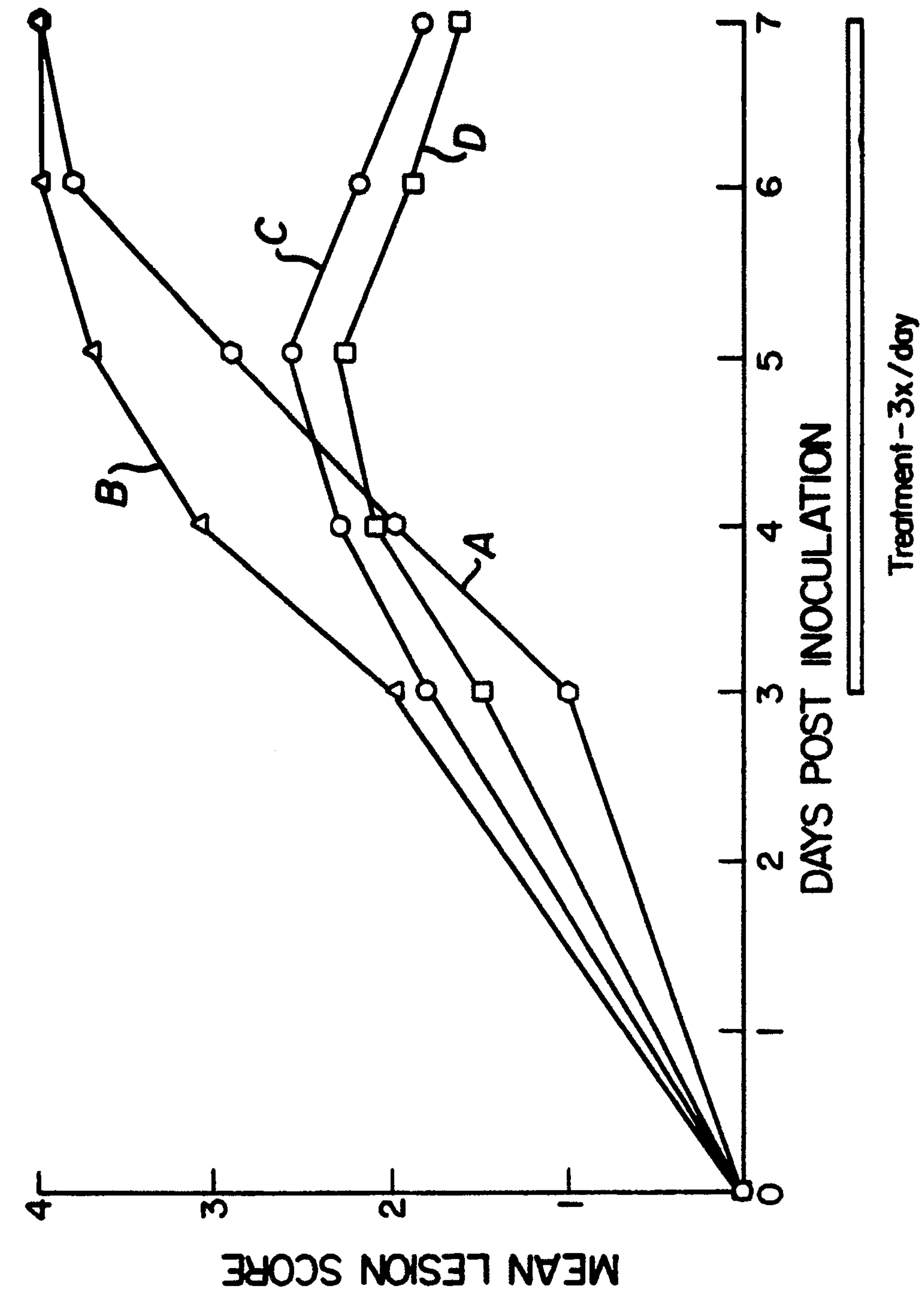
MEAN LESION SCORE
DAYS POST INOCULATION
Treatment - 3x / day
A
B
C
D
0
1
2
3
4
5
6
7